(19) Europäisches Patentamt
European Patent Office
Office européen des brevets

(11) Numéro de publication : **0 035 924**
**B1**

(12) **FASCICULE DE BREVET EUROPÉEN**

(45) Date de publication du fascicule du brevet : **03.08.83**

(21) Numéro de dépôt : **81400278.8**

(22) Date de dépôt : **23.02.81**

(51) Int. Cl.$^3$ : **C 07 D495/04**, A 61 K 31/435 // (C07D495/04, 335/00, 221/00)

(54) **Nouvelles benzothiopyrano/2,3-c/pyridines et leurs sels avec les acides, leur préparation, leur application à titre de médicaments et les compositions les renfermant.**

(30) Priorité : **06.03.80 GB 8007660**

(43) Date de publication de la demande : **16.09.81 Bulletin 81/37**

(45) Mention de la délivrance du brevet : **03.08.83 Bulletin 83/31**

(84) Etats contractants désignés : **AT BE CH DE FR IT LI LU NL SE**

(56) Documents cités : **DE A 1 811 075**

(73) Titulaire : **ROUSSEL-UCLAF**
**35, boulevard des Invalides**
**F-75007 Paris (FR)**

(72) Inventeur : **Clements-Jewery, Stephen**
**56 High Street Broad Blunsdon**
**near Swindon Wiltshire (GB)**
Inventeur : **Westwood, Robert**
**8 Fernham Road**
**Faringdon Oxon (GB)**
Inventeur : **Hairsine, Peter Wilfred**
**569 Queens Drive**
**Swindon Wiltshire SN3 1AZ (GB)**

(74) Mandataire : **Vieillefosse, Jean-Claude et al**
**ROUSSEL-UCLAF Boîte postale no. 9 102, route de Noisy**
**F-93230 Romainville (FR)**

**EP 0 035 924 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Nouvelles benzothiopyrano [2,3-c] pyridines et leurs sels avec les acides, leur préparation, leur application à titre de médicaments et les compositions les renfermant

La présente invention concerne de nouvelles benzothiopyrano [2,3-c] pyridines et leurs sels avec les acides, leur préparation, leur application à titre de médicaments et les compositions les renfermant.

La demande allemande DE-A-1 811 075 décrit des tétrahydrobenzothiopyrano [2,3-c] pyridines non substituées en position 5 par un radical phényle et présentant des propriétés analgésique et antiphlogistique.

L'invention a pour objet de nouvelles benzothiopyrano [2,3-c] pyridines ainsi que leurs sels d'addition avec les acides minéraux ou organiques, caractérisées en ce qu'elles répondent à la formule générale :

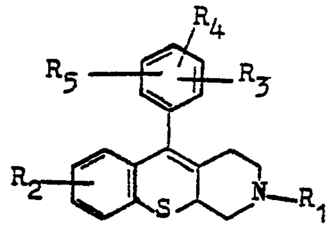

(I)

dans laquelle $R_1$ représente un atome d'hydrogène ou un radical alcoyle renfermant de 1 à 5 atomes de carbone, $R_2$ représente un atome d'hydrogène, un atome d'halogène ou un radical méthyle et $R_3$, $R_4$ et $R_5$, identiques ou différents, représentent un atome d'hydrogène, un atome d'halogène, un radical alcoyle renfermant de 1 à 5 atomes de carbone, un radical alcoxy renfermant de 1 à 3 atomes de carbone, un radical amino, un radical alcoyl ou dialcoylamino dont le radical alcoyle renferme de 1 à 3 atomes de carbone.

Dans la formule générale I et dans ce qui suit, le terme radical alcoyle renfermant de 1 à 5 atomes de carbone désigne, par exemple, un radical méthyle, éthyle, propyle, isopropyle, butyle, ou pentyle ; le terme radical alcoxy renfermant de 1 à 3 atomes de carbone désigne un radical méthoxy, éthoxy, propoxy ou isopropoxy ; le terme radical alcoyl- ou dialcoylamino dont le radical alcoyle renferme de 1 à 3 atomes de carbone désigne, par exemple, un radical méthylamino, éthylamino, diméthylamino ou diéthylamino.

Dans la formule générale I, lorsque $R_2$, $R_3$, $R_4$ et $R_5$ représentent un atome d'halogène, il s'agit de préférence, d'un atome de chlore.

Les sels d'addition avec les acides minéraux ou organiques peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, alcanesulfonique, tels que l'acide méthane sulfonique et arylsulfoniques, tels que l'acide benzène sulfonique.

Parmi les produits, objet de l'invention, on peut citer notamment les dérivés répondant à la formule I ci-dessus, ainsi que leurs sels, caractérisés en ce que dans ladite formule I, $R_1$ représente un radical méthyle et $R_2$, $R_3$, $R_4$ et $R_5$ ont la signification déjà indiquée.

Parmi les produits, objet de l'invention, on retient plus particulièrement les dérivés répondant à la formule I ci-dessus, ainsi que leurs sels, caractérisés en ce que dans ladite formule I, $R_1$ représente un radical méthyle, $R_2$ représente un atome d'hydrogène et $R_3$, $R_4$ et $R_5$ ont la signification déjà indiquée.

Parmi ces derniers, on peut citer tout particulièrement :

— la 2-méthyl 5-phényl 1,3,4,10a-tétrahydro-2H-[1] benzothiopyrano [2,3-c] pyridine et ses sels,

— la 2-méthyl 5-(4-tolyl)1,3,4,10a-tétrahydro-2H-[1] benzothiopyrano [2,3-c] pyridine et ses sels,

— la 2-méthyl 5-(2,4-diméthylphényl)1,3,4,10a-tétrahydro-2H [1] benzopyrano [2,3-c] pyridine et ses sels,

— la 2-méthyl 5-(4-diméthylaminophényl)1,3,4,10a-tétrahydro-2H [1] benzothiopyrano [2,3-c] pyridine et ses sels.

L'invention a également pour objet un procédé de préparation des produits tels que définis par la formule I ci-dessus ainsi que de leurs sels, caractérisé en ce que l'on fait réagir un organo-métallique de formule :

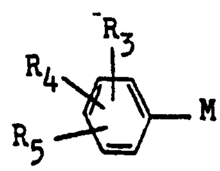

(II)

dans laquelle $R_3$, $R_4$ et $R_5$ ont la signification déjà indiquée et M représente un atome de lithium ou un radical Mg-Hal dans lequel Hal représente un atome de chlore ou de brome, ou un dérivé de cet organo-métallique, dans lequel, lorsque $R_3$ et/ou $R_4$ et/ou $R_5$ représentent un radical amino ou alkylamino, celui-ci est protégé, avec un produit de formule :

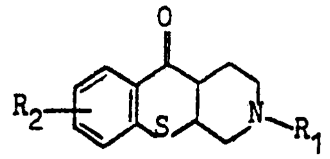

(III)

dans laquelle $R_1$ et $R_2$ ont la signification déjà indiquée, puis le cas échéant, élimine le ou les groupements protecteurs du ou des groupements amino ou alkylamino, pour obtenir un produit de formule :

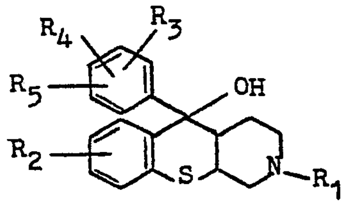

(IV)

dans laquelle $R_1$, $R_2$, $R_3$, $R_4$ et $R_5$ ont la signification déjà indiquée, puis déshydrate ce dernier pour obtenir le produit de formule I recherché que l'on peut salifier, le cas échéant.

Dans des conditions préférentielles de mise en œuvre de l'invention, le procédé de préparation ci-dessus décrit est caractérisé en ce que :

a) la réaction de l'organo métallique de formule II avec le produit de formule III est effectuée au sein d'une éther anhydre tel que l'éther éthylique ou le tétrahydrofuranne,

b) lorsque les radicaux amino et alkylamino sont protégés, le groupement protecteur est notamment un groupement trialcoylsilyl, plus particulièrement triméthylsilyl et peut être éliminé par hydrolyse, par des méthodes connues en elles-mêmes,

c) la déshydratation du produit de formule IV est effectuée à la température d'ébullition du mélange réactionnel, au moyen d'un acide fort tel que l'acide chlorhydrique ou l'acide sulfurique, ou au moyen du bisulfate de potassium, ou par chauffage dans l'hexaméthylphosphoramide.

L'invention a également pour objet un procédé de préparation des produits de formule I, tel que défini précédemment, caractérisé en ce que l'organo métallique de formule II, dans laquelle M représente un atome de lithium est préparé par action d'un produit de formule :

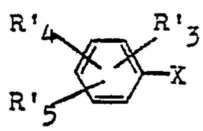

(V)

dans laquelle X représente un substituant sélectivement déplaçable par le lithium et $R'_3$, $R'_4$, et $R'_5$, identiques ou différents, représentent un atome d'hydrogène, un atome d'halogène, un radical alcoyle renfermant de 1 à 5 atomes de carbone, un radical alcoxy renfermant de 1 à 3 atomes de carbone, un radical amino ou alcoylamino protégé ou un radical dialcoylamino, les radicaux alcoyles des radicaux alcoylamino et dialcoylamino renfermant de 1 à 3 atomes de carbone, avec un alcoyl lithium suivi, si désiré, d'une hydrolyse des groupements protecteurs des radicaux amino ou alcoylamino.

Dans des conditions préférentielles de mise en œuvre de l'invention :

a) l'organo métallique de formule II est préparé *in situ* ;

b) les radicaux amino et alcoylamino sont protégés de la manière décrite précédemment ;

c) X est un atome de chlore, de brome ou d'iode et, lorsque $R'_3$, $R'_4$ et $R'_5$ sont eux-mêmes des halogènes, X est un halogène plus facilement déplacé ;

d) l'alcoyl lithium est le butyl lithium ;

e) l'hydrolyse éventuelle des groupements protecteurs est effectuée par des méthodes connues en elles-mêmes.

Dans le procédé de l'invention, l'hydrolyse du groupement protecteur des radicaux amino et alcoylamino peut intervenir, selon les cas, avant ou après l'action du produit de formule II, sur le produit de formule III.

Les produits, objet de la présente invention, possèdent de très intéressantes propriétés pharmacologiques ; ils sont doués notamment de remarquables propriétés antidépressives et neuroleptiques. Ils présentent, en outre, l'avantage d'être dénués des propriétés sédatives, relaxantes musculaires et anticholinergiques usuelles dans ce genre de produits antidépresseurs tricycliques et des effets extrapyramidaux des neuroleptiques.

Les propriétés pharmacologiques des produits sont illustrées plus loin dans la partie expérimentale.

Ces propriétés justifient l'utilisation des nouvelles benzothiopyrano [2,3-c] pyridines ainsi que de

leurs sels pharmaceutiquement acceptables, à titre de médicaments.

La présente invention a ainsi également pour objet l'application à titre de médicaments des nouvelles benzothiopyrano [2,3-c] pyridines telles que définies par la formule générale I, ainsi que de leurs sels d'addition avec les acides pharmaceutiquement acceptables.

Parmi les médicaments, objet de l'invention, on retient notamment les médicaments caractérisés en ce qu'ils sont constitués par les nouvelles benzothiopyrano [2,3-c] pyridines répondant à la formule générale I dans laquelle $R_1$ représente un radical méthyle et $R_2$, $R_3$, $R_4$ et $R_5$ ont la signification déjà indiquée, ainsi que leurs sels d'addition avec les acides pharmaceutiquement acceptables.

Parmi ces derniers, on retient tout particulièrement ceux répondant à la formule I, caractérisés en ce que $R_1$ représente un radical méthyle, $R_3$, $R_4$ et $R_5$ ont la signification déjà indiquée et $R_2$ représente un atome d'hydrogène, ainsi que leurs sels d'addition avec les acides pharmaceutiquement acceptables.

Parmi les médicaments préférés de l'invention, on retient tout particulièrement :

— la 2-méthyl 5-phényl 1,3,4,10a-tétrahydro-2H-[1] benzothiopyrano [2,3-c] pyridine et ses sels,

— la 2-méthyl 5-(4-tolyl)1,3,4,10a-tétrahydro-2H-[1] benzothiopyrano [2,3-c] pyridine et ses sels,

— la 2-méthyl 5-(2,4-diméthylphényl)1,3,4,10a-tétrahydro-2H [1] benzothiopyrano [2,3-c] pyridine et ses sels,

— la 2-méthyl 5-(4-diméthylaminophényl)1,3,4,10a-tétrahydro 2H-[1] benzothiopyrano [2,3-c] pyridine et ses sels.

Ces médicaments trouvent, par exemple, leur emploi dans le traitement des troubles psychiques, des troubles du comportement et des troubles caractériels.

La dose usuelle, variable selon le produit utilisé, le sujet traité et l'affection en cause peut être, par exemple, de 2 mg à 2 g par jour, par voie orale.

L'invention a enfin pour objet les compositions pharmaceutiques qui renferment au moins un dérivé précité ou l'un de ses sels d'addition avec les acides pharmaceutiquement acceptables à titre de principe actif.

A titre de médicaments, les dérivés répondant à la formule I et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les capsules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de formule III, lorsqu'ils ne sont pas connus, peuvent être préparés par un procédé analogue à celui décrit dans CH-A-543.530 ; ils peuvent également être préparés selon les indications figurant dans GB-A-1 252 131 ou 1 252 132.

Il va être donné maintenant à titre non limitatif, des exemples de mise en œuvre de l'invention.

Exemple I : Chlorhydrate 2-méthyl-5-phényl-1,3,4,10a-tétrahydro-2H [1]-benzothiopyrano [2,3-c] pyridine.

Stade A : 2-méthyl-5-phényl-1,3,4,4a,5,10a-hexahydro-2H-[1] benzothiopyrano [2,3-c] pyridine-5-ol

On dissout 40 g de 1,2,3,4,4a,10a-hexahydro-2-méthyl 5H-[1] benzothiopyrano [2,3-c] pyridin-5-one dans 2 litres d'éther éthylique, refroidit la solution à 5 °C, puis ajoute goutte à goutte, 220 ml d'une solution 2M de phényl lithium dans l'éther, en 30 minutes, en maintenant la température inférieure à + 10 °C. Lorsque l'addition est terminée on laisse la température remonter à 20 °C, puis agite pendant 30 minutes. On ajoute goutte à goutte 3 litres d'eau puis agite vigoureusement pendant 2 heures la suspension obtenue. On filtre, lave le filtre à l'eau puis à l'éther, le sèche et obtient 20,5 g de produit attendu. F = 176-181 °C.

Stade B : Chlorhydrate 2-méthyl-5-phényl-1,3,4,10a-tétrahydro-2H [1]-benzothiopyrano [2,3-c] pyridine

On ajoute 21,4 g de produit obtenu au stade A dans une solution renfermant 42,8 $cm^3$ d'acide chlorhydrique concentré et 85,6 $cm^3$ d'eau puis porte au reflux pendant 1 heure sous agitation. On refroidit, filtre les cristaux formés, les sèche et obtient 19,3 g de produit attendu. F = 234-240 °C.

La 1,2,3,4,4a,10a-hexahydro-2-méthyl-5H [1] Benzothiopyrano/2,3-c/pyridin-5-one a été préparée comme suit :

a) Iodure de 4-méthoxycarbonyl-1-méthylpyridinium.

On agite au reflux pendant 5 heures une solution renfermant 700 g d'isonicotinate de méthyle et 1,4 kg d'iodure de méthyle dans 2 litres de méthanol. Après refroidissement, on filtre le solide formé, le

lave à l'éther et le sèche. On obtient 1,385 kg de produit attendu.

b) 1,2,3,6-tétrahydro-1-méthylisonicotinate de méthyle.

On agite puis refroidit à 5 °C une suspension de 1,385 kg de produit obtenu précédemment, dans 25 litres de méthanol sec. On ajoute en petites quantités sur une période d'1 heure à 5-10 °C, 700 g de borohydrure de sodium. On agite ensuite la solution obtenue à température ambiante pendant 1 heure, puis concentre à environ 5 litres. On ajoute 25 litres d'eau, puis 2 kg de carbonate de sodium et extrait la solution obtenue au chloroforme. On sèche la phase organique, évapore le solvant, puis rectifie l'huile obtenue et obtient 583 g de produit attendu. Eb (1 mm Hg = 133,322 Pa) = 78 °C.

c) 1-méthyl 3-(phénylthio)-isonipécotinate de méthyle.

On porte au reflux sous gaz inerte pendant 6 heures une solution de 583 g de produit obtenu au stade b, 373,5 $cm^3$ de benzènethiol et 72,8 $cm^3$ de pipéridine dans 7,28 litres de méthanol. On refroidit puis concentre à environ 2 litres. On ajoute 8 litres d'eau, et extrait la solution obtenue à l'acétate d'éthyle. On lave la phase organique avec une solution aqueuse 1 N. d'hydroxyde de sodium, puis à l'eau, sèche et concentre à sec. On obtient 813 g de produit attendu utilisé tel quel pour le stade suivant.

d) 1,2,3,4,4a,10a-hexahydro-2-méthyl-5H-[1] benzothiopyrano [2,3-c] pyridine-5-one.

On agite à 120 °C sous atmosphère inerte 3,43 kg d'acide tétraphosphorique. On cesse ensuite le chauffage, puis ajoute en 30 minutes 327 g de 1-méthyl-3-(phénylthio)-isonipécotinate de méthyle. La température monte à 135 °C et est maintenue à cette valeur pendant 4 heures. La solution obtenue est refroidie à 100 °C, versée dans 30 litres d'eau glacée sous vigoureuse agitation, jusqu'à dissolution complète. On ajoute lentement en refroidissant de manière à maintenir la température inférieure à 20 °C, une solution aqueuse à 50 % en poids d'hydroxyde de sodium, jusqu'à ce que le pH atteigne 7. La solution aqueuse obtenue est extraite à l'acétate d'éthyle. Les phases organiques sont lavées à l'eau, séchées et concentrées à sec. Le résidu obtenu est distillé et l'on obtient 117,3 g de produit attendu. Eb (0,2 mm Hg = 26,66 Pa) = 156-162 °C.

Exemples 2 à 24

En utilisant une méthode analogue à celle décrite à l'exemple I, les composés décrits dans le tableau 1 qui suit, ont été préparés.

| Exemple No | $R_1$ | $R_2$ | 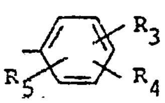 | F °C HCl |
|---|---|---|---|---|
| 2 | $CH_3$ | H | 4-chlorophényl | 215-8 |
| 3 | $CH_3$ | H | 4-méthylphényl | 243-5 |
| 4 | $CH_3$ | H | 4-méthoxyphényl | 218-220 |
| 5 | $CH_3$ | H | 3-chlorophényl | 201-3 |
| 6 | $CH_3$ | H | 4-(diméthylamino)phényl | 243-5 |
| 7 | $CH_3$ | H | 3-méthylphényl | 204-6 |
| 8 | $CH_3$ | H | 3,4-dichlorophényl | 214-6 |
| 9 | $CH_3$ | H | 4-éthylphényl | 230-2 |
| 10 | $CH_3$ | H | 2-méthylphényl | 246-8 |
| 11 | $CH_3$ | H | 4-n-propoxyphényl | 197-9 |
| 12 | $C_2H_5$ | H | phényl | 211-3 |
| 13 | $CH_3$ | 7-Cl | phényl | 221-5 |
| 14 | $CH_3$ | H | 3,4-diméthylphényl | 208-10 |
| 15 | $CH_3$ | H | 4-aminophényl | 230-2 |
| 16 | $CH_3$ | 8-$CH_3$ | phényl | 205-7 |
| 17 | H | H | phényl | 259-7 |
| 18 | $CH_3$ | 9-$CH_3$ | phényl | 221-8 |
| 19 | $CH_3$ | H | 2,4-diméthylphényl | 206-9 |
| 20 | $CH_3$ | 9-Cl | phényl | 213-5 |
| 21 | $CH_3$ | 8-Cl | phényl | 199-201 |
| 22 | $CH_3$ | 7-$CH_3$ | phényl | 206-7 |
| 23 | $CH_3$ | H | 2,6-diméthylphényl | 231-3 |
| 24 | $CH_3$ | H | 2,4,6-triméthylphényl | 237-40 |

| | | | | | | | |
|---|---|---|---|---|---|---|---|
| | | | | | a | b | c |
| | | | Calculé/trouvé | | | | |
| Exemple No | % C | % H | % N | % Cl | % S | Masse moléc. | Formule |
| 2 | 62.64 | 5.26 | 3.84 | 19.46 | 8.80 | 327 | $C_{19}H_{19}NSCl_2$ |
| | 62.15 | 5.20 | 3.76 | 19.75 | 8.75 | | |
| 3 | 69.85 | 6.45 | 4.07 | 10.31 | 9.32 | 307 | $C_{20}H_{22}NSCl$ |
| | 69.19 | 6.34 | 3.93 | 10.47 | 9.12 | | |
| 4 | 66.74 | 6.16 | 3.89 | 9.85 | 8.91 | 322 | $C_{20}H_{22}CNSCl$ |
| | 66.56 | 6.12 | 3.76 | 9.75 | 8.69 | | |
| 5 | 62.64 | 5.26 | 3.84 | 19.46 | 8.80 | 327 | $C_{19}H_{19}NSCl_2$ |
| | 61.90 | 5.20 | 3.78 | 19.62 | 8.68 | | |
| 6 | 67.63 | 6.76 | 7.51 | 9.51 | 8.60 | 336 | $C_{21}H_{25}N_2SCl$ |
| | 66.96 | 6.78 | 7.41 | 9.60 | 8.48 | | |
| 7 | 69.85 | 6.45 | 4.07 | 10.31 | 9.32 | 307 | $C_{20}H_{22}NSCl$ |
| | 69.48 | 6.46 | 3.97 | 10.40 | 9.32 | | |
| 8 | 57.23 | 4.55 | 3.51 | 26.67 | 8.04 | 361 | $C_{19}H_{18}NSCl_3$ |
| | 57.01 | 4.49 | 3.47 | 26.80 | 7.95 | | |
| 9[a] | 70.47 | 6.76 | 3.91 | 9.90 | 8.96 | 321 | $C_{21}H_{24}NSCl$ |
| | 68.99 | 6.59 | 3.78 | 11.51 | 8.82 | | |
| 10 | 69.85 | 6.45 | 4.07 | 10.31 | 9.32 | 307 | $C_{20}H_{22}NSCl$ |
| | 69.86 | 6.37 | 4.07 | 10.75 | 9.43 | | |
| 11 | 68.11 | 6.76 | 3.61 | 8.26 | 9.14 | 351 | $C_{22}H_{23}NOSCl$ |
| | 68.07 | 6.72 | 3.58 | 8.24 | — | | |
| 12 | 69.85 | 6.45 | 4.07 | 10.31 | 9.32 | 307 | $C_{20}H_{22}NSCl$ |
| | 69.37 | 6.39 | 4.02 | — | — | | |
| 13 | 62.64 | 5.26 | 3.84 | 19.46 | 8.80 | 327 | $C_{19}H_{19}NSCl_2$ |
| | 62.87 | 5.32 | 3.79 | 19.15 | — | | |
| 14 | 70.47 | 6.76 | 3.91 | 9.90 | 8.96 | 321 | $C_{21}H_{24}NSCl$ |
| | 70.21 | 6.74 | 3.80 | 10.20 | — | | |
| 15 | 59.84 | 5.82 | 7.34 | 18.59 | 8.51 | 302 | $C_{19}H_{12}N_2SCl_2$ |
| | 59.45 | 5.80 | 7.02 | — | 8.17 | | |
| 16 | 69.85 | 6.45 | 4.07 | 10.31 | 9.32 | 307 | $C_{20}H_{22}NSCl$ |
| | 69.29 | 6.39 | 4.01 | 10.42 | 9.24 | | |
| 17 | 68.45 | 5.74 | 4.44 | 11.22 | 10.15 | 279 | $C_{18}H_{18}NClS$ |
| | 68.25 | 5.89 | 4.36 | 10.83 | 9.97 | | |
| 18[a] | 69.85 | 6.45 | 4.07 | 10.31 | 9.32 | 307 | $C_{20}H_{22}NSCl$ |
| | 68.26 | 6.37 | 3.97 | 10.07 | 9.10 | | |
| 19 | 70.47 | 6.76 | 3.91 | 9.90 | 8.96 | 321 | $C_{21}H_{24}NSCl$ |
| | 70.47 | 6.76 | 3.90 | 9.94 | 8.96 | | |
| 20 | 62.64 | 5.26 | 3.84 | 19.46 | 8.80 | 327 | $C_{19}H_{19}NSCl$ |
| | 62.05 | 5.21 | 3.80 | 19.42 | 8.82 | | |
| 21 | 62.64 | 5.26 | 3.84 | 19.46 | 8.80 | 327 | $C_{19}H_{19}NSCl$ |
| | 61.60 | 5.26 | 3.75 | 19.55 | 8.64 | | |
| 22 | 69.85 | 6.45 | 4.07 | 10.31 | 9.32 | 307 | $C_{20}H_{22}NSCl$ |
| | 68.36 | 6.59 | 3.90 | 10.10 | 9.21 | | |
| 23 | 70.47 | 6.76 | 3.91 | 9.90 | 8.96 | 321 | $C_{21}H_{24}NSCl$ |
| | 70.07 | 6.66 | 3.85 | 9.69 | 8.96 | | |
| 24 | 71.04 | 7.05 | 3.76 | 9.53 | 8.62 | 335 | $C_{22}H_{26}NSCl$ |
| | 70.59 | 7.06 | 3.90 | 9.89 | 8.44 | | |

a = Calculé pour 0,5 M. $H_2O$ b = Base libre c = Chlorhydrate

Exemple 25 : Chlorhydrate de 5-(4-aminophényl)2-méthyl-1,3,4,10a-tétrahydro-2H-[1] benzothiopyrano [2,3-c] pyridine.

Stade A : 5-(4-aminophényl)-2-méthyl-1,3,4,4a,5,10a-hexahydro 2H [1]-benzothiopyrano [2,3-c] pyridine-5-ol.

On dissout 3 g de parachloro-N,N-bis(triméthylsilyl)aniline, (préparé comme décrit dans J.C.S, 1966, 1706) dans 30 $cm^3$ d'éther éthylique. On ajoute 12 $cm^3$ d'une solution 1,6 M. de n-butyl lithium dans l'hexane. Une heure plus tard on ajoute 1,5 g de 1,2,3,4,4a,10a-hexahydro-2-méthyl-2H-[1]-benzothiopyrano [2,3-c] pyridine-5-one et agite le mélange obtenu à température ambiante pendant 2 heures. On ajoute de l'eau, maintient l'agitation pendant 2 heures, sépare la phase organique, la sèche et évapore le solvant. Le résidu est purifié par chromatographie sur silice en éluant au chloroforme à 5 % de méthanol, et obtient 1 g de produit attendu. F = 169 °C.

Stade B : Chlorhydrate de 5-(4-aminophényl)-2-méthyl-1,3,4,10a-tétrahydro-2H-[1] benzothiopyrano [2,3-c] pyridine.

On porte au reflux pendant 2 heures un mélange de 1 g de produit obtenu au stade A, dans l'acide chlorhydrique 5,5 N. On ajoute à la solution obtenue du bicarbonate de sodium jusqu'à l'obtention d'un pH basique, puis extrait au chloroforme. On évapore le solvant, dissout le résidu dans l'acétate d'éthyle et traite par une solution d'acide chlorhydrique dans l'acétate d'éthyle, essore les cristaux formés, les sèche, et obtient 0,8 g de produit attendu. F = 230-232 °C.

Exemple 26

On a préparé des comprimés renfermant :

| | |
|---|---|
| — Chlorhydrate de méthyl-5-(2,4-diméthylphényl)1,3,4,10a tétrahydro-2H-[1] benzothiopyrano [2,3-c] pyridine | 25 mg |
| — excipient (lactose, talc, amidon, stéarate de magnésium) qs. pour 1 comprimé | 150 mg. |

Activité Pharmacologique

1. Liaison de [3H] spiroperidol® aux récepteurs « neuroleptiques » sur des membranes du striatum et du cortex frontal des rats.

La liaison de 3H spiroperidol sur les membranes du cortex frontal du rat présente une composante principalement sérotoninergique, alors qu'au niveau du striatum la liaison est du type dopaminergique (Leysen, J.E et al Nature 272, 168-171 (1978)). La capacité de ces composés à déplacer ce marqueur radioactif fixé sur des récepteurs du cortex frontal donne une bonne corrélation avec leurs effets sur d'autres modèles d'activité sérotoninergique, aussi bien pour des agonistes que pour des antagonistes. De même, les activités obtenues au niveau des membranes du striatum donne une bonne indication de l'effet antidopaminergique des composés neuroleptiques.

Matériel et méthode

Ligand radioactif : Le [phényl-4(n)-3H] spiroperidol® (Radiochemical centre, TRK 570) 21 Ci/mmol est utilisé, conservé en solution dans l'éthanol (250 µCi/250 µl). Cette solution est diluée dans de l'éthanol (250 µCi/1 ml) et cette solution stock est diluée dans un tampon (3,2/10 000). L'essai est réalisé avec 0,1 ml de cette solution (le volume final du milieu de l'incubation étant de 2 ml).

= 0,19 nM de [3H] spiroperidol®
= 0,08 µCi/par échantillon

Tampon : le tampon utilisé est un tampon Tris HCL 50 mM pH 7,6 contenant les produits suivants

| | |
|---|---|
| — NaCL | 120 mM |
| — KCl | 5 mM |
| — $MgCl_2$ | 1 mm |
| — $CaCl_2$ | 2 mM |
| — Pargyline | 10 µM |
| — Acide ascorbique | 0,1 % |

Préparation des membranes

Le cortex frontal et le striatum sont disséqués à partir du cerveau des 4 rats mâles (CFHB) selon la méthode de J. GLOWINSKI et L.L. IVERSEN (Eur. J. PHARMACOL 49, 201-202-(1978)). Le cortex frontal étant considéré comme le tissu cortical correspondant à la partie « C » (voir référence).

Les animaux sont sacrifiés par décapitation et les tissus après dissection sont homogénisés à 0 °C dans 20 volumes de sucrose 0,32 M avec un homogéniseur Teflon/verre. L'homogénat est centrifugé à 1 000 g pendant 10 minutes et le surnageant est recentrifugé à 30 000 g pendant 30 minutes. Le surnageant est éliminé et le culot est remis en suspension par homogénisation dans 30 volumes de tampon à 0 °C.

Liaison non spécifique

La liaison non spécifique est évaluée en ajoutant du butaclamol froid (1 µM) aux échantillons blancs.

On utilise le butaclamol® en solution aqueuse à 20 μM.

Les Composés à étudier

Les solutions sont préparées dans de l'eau distillée à une concentration 20 fois supérieure à celle de la concentration finale par échantillon.

Protocole

— 1,3 ml de tampon
— 0,5 ml de suspension membranaire
— 0,1 ml d'eau distillée ou solution de composé
— 0,1 ml de [3H] spiroperidol®

Les tubes sont conservés dans la glace. Après l'addition de [3H] spiroperidol® les échantillons sont incubés à 37 °C pendant 10 minutes. Les membranes sont séparées par filtration sur des filtres Whatman® GF/C sous vide, puis sont rincées par 2 × 10 ml de tampon à 0 °C. Les filtres sont séchés à 80 °C et la radioactivité est comptée dans 5 ml d'éconofluor® (NEN). Tous les échantillons sont réalisés en triple.

Les résultats du déplacement du [3H] spiroperidol® fixé sur les récepteurs du cortex frontal (test A) et des membranes striatales (test B) sont rassemblés dans le tableau II ci-dessous. Les résultats sont exprimés en $CI_{50}$ (nM).

2. Liaison aux récepteurs α-noradrénergiques sur les membranes du cortex du rat à l'aide du [3H] WB 4101.

Le site récepteur α-noradrénergique au niveau du cerveau des mammifères peut être marqué à l'aide de [3H] WB 4101 (2-([2'6'-diméthoxy] phénoxyméthylamino)méthyl-benzo dioxan), qui est un antagoniste α-adrénergique puissant (Peroutka S.S. et al. Neuropharm. 16 549-556 (1977)). L'affinité des neuroleptiques et des thymoleptiques pour les sites marqués par le [3H] WB 4101 est en étroite corrélation avec leur capacité à antagoniser la mortalité induite par la noradrénaline et l'adrénaline chez le rat, ce qui confirme que l'aptitude à déplacer le [3H] WB 4101 de ces sites de liaison donne une bonne indication de leur effet antagoniste au niveau du récepteur α-adrénergique *in vivo*. Les affinités relatives des composés pour les sites de liaison au [3H] WB 4101 peuvent servir d'index pour évaluer leur tendance à provoquer des effets secondaires autonomes sur le système nerveux, tel l'hypertension et la sédation.

Matériel et méthode

Ligand radioactif : le [3H] WB 4101 (Radiochemical Centre TRK 579) 20 Ci/mmol est utilisé à une dilution 1/10 000 dans un tampon. 0,1 ml de cette solution est ajouté à 2 ml (volume total) de la solution à tester.

= 0,25 nM de [3H] WB 4101
= 0,01 μCi par échantillon

Préparation du Tissu

Un rat mâle adulte (CFHB) est sacrifié par décapitation. Le cerveau est enlevé et placé dans une solution de sucrose (0,32 M) à 0 °C. Le tronc cérébral et le cervelet sont éliminés et le cerveau antérieur est homogénisé dans 20 volumes de sucrose (0,32 M) à 0 °C à l'aide d'un homogéniseur Teflon/verre. L'homogénat est centrifugé à 1 000 g pendant 10 minutes et le culot est éliminé et le surnageant est recentrifugé à 30 000 g pendant 30 minutes. Le surnageant est éliminé et le culot est remis en suspension par homogénisation dans 50 volumes de tampon Tris HCl mM pH 7,5.

Liaison non spécifique

La liaison non spécifique est évaluée en ajoutant 100 μM de noradrénaline froide aux échantillons blancs. On utilise une solution extemporanée de noradrénaline à 2 mM dans de l'acide ascorbique 0,1 %.

Composés à tester

Les solutions sont préparées dans du tampon à une concentration 20 fois supérieure à celle de la concentration finale par échantillon.

Protocole

1,3 ml de tampon

0,5 ml de suspension membranaire
0,1 ml de tampon ou solution de composé
0,1 ml de [3H] WB 4101

Les tubes sont conservés dans la glace. Après l'addition du [3H] ligand, les échantillons sont incubés à 25 °C pendant 15 minutes. Les membranes sont séparées par filtration sur des filtres Whatman® GF/C sous vide, puis sont rincées par 2 × 10 ml de tampon à 0 °C. Les filtres sont séchés à 80 °C et la radioactivité est comptée dans 5 ml d'econofluor® (NEN). Tous les échantillons sont réalisés en triple.

Les résultats du déplacement du [3H] WB 4101 fixé sur les récepteurs des membranes du cortex du rat sont rassemblés dans le tableau II. (Test C). Les résultats sont exprimés en $CI_{50}$ (nM).

3. Liaison aux récepteurs muscariniques sur des membranes du cerveau antérieur du rat à l'aide du [3H] QNB.

Des sites de liaison à haute affinité pour le [3H] QNB (quinuclidinyl benzilate) sont présents dans des homogénats du cerveau de rat. Les caractéristiques de ces sites de liaison ressemblent à ceux des récepteurs muscariniques cholinergiques. Les agonistes et antagonistes muscariniques déplacent la liaison spécifique du [3H] QNB avec une intensité qui est en rapport avec la puissance de leur effet pharmacologique. Les effets anticholinergiques des antidépresseurs et neuroleptiques peuvent avoir des conséquences pour leur utilisation chez l'homme car ils entraînent une mauvaise tolérance de ces composés (Snyder et al. Arch. gen. Psychiatr. 34(2) 326-329 (1977)).

## Matériel et méthode

Ligand radioactif : (3-[3H]) Quinuclidinyl benzilate (Amersham TRK 506) 16,4 Ci/mmol est utilisé dilué dans du tampon (1/10 000). L'essai est réalisé avec 0,1 ml de cette solution (le volume final du milieu de l'incubation étant de 2 ml).

= 0,3 nM de [3H] QNB
= 0,01 µCi par échantillon

## Préparation du tissu

Un rat mâle adulte (CFHB) est sacrifié par décapitation. Le cerveau est enlevé et placé dans une solution de sucrose (0,32 M) à 0 °C. Le tronc cérébral et le cervelet sont éliminés et le cerveau antérieur est homogénisé dans 20 volumes de sucrose (0,32 M) à 0 °C à l'aide d'un homogéniseur Teflon/verre. L'homogénat est centrifugé à 1 000 g pendant 10 minutes, le culot est éliminé et le surnageant est recentrifugé à 30 000 g pendant 30 minutes. Le surnageant est éliminé et le culot est remis en suspension par homogénisation dans 50 volumes de tampon Tris HCl 50 mM pH 7,5.

## Liaison non spécifique

La liaison non spécifique est évaluée en ajoutant 100 µM d'oxotrémorine froide aux échantillons blancs. On utilise une solution d'oxotrémorine dans du tampon (2 mM).

## Composés à tester

Les solutions sont préparées dans du tampon à une concentration 20 fois supérieure à celle de la concentration finale par échantillon.

## Protocole

1,3 ml de tampon
0,5 ml de suspension membranaire
0,1 ml de tampon ou solution de composé
0,1 ml de [3H] QNB

Les tubes sont conservés dans la glace. Après l'addition de [3H] QNB les échantillons sont incubés à 25 °C pendant une heure. Les membranes sont séparées par filtration sur des filtres Whatman GF/C sous vide, puis sont rincées par 2 × 10 ml de tampon à 0 °C. Les filtres sont séchés à 80 °C et la radioactivité est comptée dans 5 ml d'éconofluor® (NEN). Tous les échantillons sont réalisés en triple.

Les résultats du déplacement du [3H] QNB fixé sur les récepteurs des membranes du cerveau antérieur du rat, sont rassemblés dans le tableau II. (Test D). Les résultats sont exprimés en $CI_{50}$ (nM).

4. L'antagonisme de l'hypothermie induite par l'apomorphine chez la souris

Alors que l'antagonisme de l'hypothermie provoquée chez la souris par de faibles doses de l'apomorphine est caractéristique des neuroleptiques classiques, l'hypothermie induite par de fortes doses de cette substance est spécifiquement renversée par des antidépresseurs (Puech et Al). Voir le texte GB.

Des groupes de 10 souris mâles CD/1 (16-22 g) sont traités par voie intrapéritonéale avec le solvant ou le composé à étudier 30 minutes avant l'injection du chlorhydrate d'apomorphine par voie sous cutanée (16 mg/kg).

La température rectale est enregistrée 30 minutes plus tard à l'aide d'un thermomètre à lecture digitale.

Les $DE_{50}$ (mg/kg i. p.) pour l'antagonisme de l'hypothermie induite par l'apomorphine HCl chez la souris sont rassemblés dans le tableau 2. (Test E).

| Composés de l'exemple | $CI_{50}$ (nM) | | | | |
|---|---|---|---|---|---|
| | Test A | Test B | Test C | Test D | Test E |
| 1 | 250 | 1 230 | 394 | 14 000 | 30 |
| 2 | 602 | 2 600 | 692 | > 100 000 | > 40 |
| 3 | 25 | 154 | 29 | 73 600 | 20 |
| 4 | 131 | 955 | 120 | 62 000 | 15 |
| 5 | 427 | 3 630 | 1 950 | 16 200 | > 40 |
| 6 | 112 | 309 | 100 | > 100 000 | — |
| 7 | 251 | 2 140 | 813 | 8 710 | > 40 |
| 8 | 537 | 3 310 | 631 | > 100 000 | — |
| 9 | 50 | 490 | 59 | > 100 000 | > 40 |
| 10 | 105 | 1 230 | 813 | 8 130 | 80 |
| 11 | 1 250 | 9 330 | 6 760 | > 100 000 | 22 |
| 12 | 398 | 3 500 | 502 | 19 030 | — |
| 13 | 1 480 | 6 300 | 355 | 47 900 | > 40 |
| 14 | 155 | 1 469 | 502 | > 100 000 | 30 |
| 15 | 158 | 1 300 | 372 | 7 940 | 5 |
| 16 | 708 | 6 310 | 1 450 | 29 500 | — |
| 17 | 624 | 6 760 | 676 | 19 500 | 35 |
| 18 | 174 | 1 000 | 794 | 38 900 | > 40 |
| 19 | 32 | 209 | 23 | 42 700 | — |
| 20 | 304 | 751 | 1 050 | 67 700 | — |
| 21 | 1 140 | 7 010 | 3 020 | 58 900 | > 100 |
| 22 | 2 450 | 9 775 | 12 500 | > 100 000 | > 100 |
| 23 | 331 | 4 520 | 617 | 4 370 | > 40 |
| 24 | 308 | 4 330 | 617 | 1 180 | > 40 |

Les résultats des tests A et B montrent l'importante affinité des composés testés pour les récepteurs neuroleptiques. Cependant, ces composés ne montrent pas d'effets extrapyramidaux classiques pour ce type de composés. Les résultats du test C montrent que quelques uns des composés testés ont de légers effets secondaires sédatifs et autonomes, tandis que ceux du test D montrent la faible activité anticholinergique. Les résultats du test E montrent l'activité anti-dépressive des composés testés.

**Revendications** (pour les Etats contractants : BE, CH, DE, FR, IT, LI, LU, NL, SE)

1. Nouvelles benzothiopyrano [2,3-c] pyridines ainsi que leurs sels d'addition avec les acides minéraux ou organiques, caractérisées en ce qu'elles répondent à la formule générale :

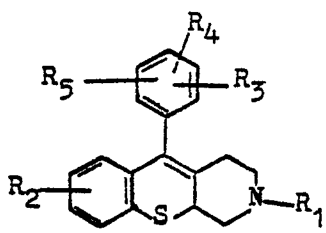

(I)

dans laquelle $R_1$ représente un atome d'hydrogène ou un radical alcoyle renfermant de 1 à 5 atomes de

carbone, $R_2$ représente un atome d'hydrogène, un atome d'halogène ou un radical méthyle et $R_3$, $R_4$ et $R_5$, identiques ou différents, représentent un atome d'hydrogène, un atome d'halogène, un radical alcoyle renfermant de 1 à 5 atomes de carbone, un radical alcoxy renfermant de 1 à 3 atomes de carbone, un radical amino, un radical alcoyl ou dialcoylamino dont le radical alcoyle renferme de 1 à 3 atomes de carbone.

2. Dérivés répondant à la formule I de la revendication 1 ainsi que leurs sels, caractérisés en ce que dans ladite formule I, $R_1$ représente un radical méthyle et $R_2$, $R_3$ $R_4$ et $R_5$ ont la signification déjà indiquée.

3. Dérivés répondant à la formule I de la revendication 1 ainsi que leurs sels, caractérisés en ce que dans ladite formule I, $R_1$ représente un radical méthyle, $R_2$ représente un atome d'hydrogène et $R_3$, $R_4$ et $R_5$ ont la signification déjà indiquée.

4. L'un quelconque des dérivés répondant à la formule I de la revendication 1, dont les noms suivent :

— la 2-méthyl 5-phényl 1,3,4,10a-tétrahydro-2H-[1] benzothiopyrano [2,3-c] pyridine et ses sels,

— la 2-méthyl 5-(4-tolyl)1,3,4,10a-tétrahydro-2H-[1] benzothiopyrano [2,3-c] pyridine et ses sels,

— la 2-méthyl 5-(2,4-diméthylphényl)1,3,4,10a-tétrahydro-2H [1] benzothiopyrano [2,3-c] pyridine et ses sels,

— la 2-méthyl 5-(4-diméthylaminophényl)1,3,4,10a-tétrahydro-2H [1] benzothiopyrano [2,3-c] pyridine et ses sels.

5. Procédé de préparation des dérivés répondant à la formule générale I de la revendication 1, ainsi que de leurs sels, caractérisé en ce que l'on fait réagir un organo métallique de formule :

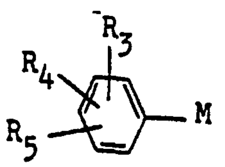

(II)

dans laquelle $R_3$, $R_4$ et $R_5$ ont la signification indiquée à la revendication 1, et M représente un atome de lithium ou un radical Mg-Hal dans lequel Hal représente un atome de chlore ou de brome, ou un dérivé de cet organo-métallique, dans lequel le ou les éventuels radicaux amino ou alkylamino sont protégés, avec un produit de formule :

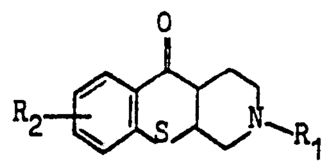

(III)

dans laquelle $R_1$ et $R_2$ ont la signification indiquée à la revendication 1, puis le cas échéant, élimine le ou les groupements protecteurs du ou des groupements amino ou alkylamino, pour obtenir un produit de formule :

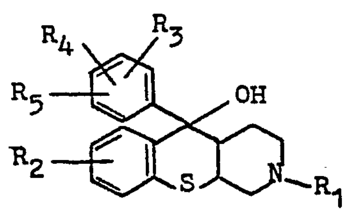

(IV)

dans laquelle $R_1$, $R_2$, $R_3$, $R_4$ et $R_5$ ont la signification déjà indiquée, puis déshydrate ce dernier pour obtenir le produit de formule I recherché que l'on peut salifier, le cas échéant.

6. Procédé selon la revendication 5, caractérisé en ce que :

a) la réaction de l'organo métallique de formule II avec le produit de formule III est effectuée au sein d'un éther anhydre ;

b) la déshydratation du produit de formule IV est effectuée à la température d'ébullition du mélange réactionnel, au moyen d'un acide fort, ou au moyen du bisulfate de potassium, ou par chauffage dans l'hexaméthylphosphoramide.

7. Procédé selon la revendication 5, caractérisé en ce que l'organo métallique de formule II, dans laquelle M représente un atome de lithium est préparé par action d'un produit de formule :

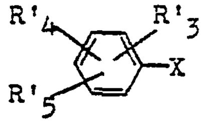

(V)

dans laquelle X représente un substituant sélectivement déplaçable par le lithium et R'$_3$, R'$_4$, et R'$_5$, identiques ou différents, représentent un atome d'hydrogène, un atome d'halogène, un radical alcoyle renfermant de 1 à 5 atomes de carbone, un radical alcoxy renfermant de 1 à 3 atomes de carbone, un radical amino ou alcoylamino protégé ou un radical dialcoylamino, les radicaux alcoyles des radicaux alcoylamino et dialcoylamino renfermant de 1 à 3 atomes de carbone, avec un alcoyl lithium suivie, si désiré, d'une hydrolyse des groupements protecteurs des radicaux amino ou alcoylamino.

8. Procédé selon la revendication 7, caractérisé en ce que l'organo métallique de formule II est préparé *in situ*.

9. Médicaments, caractérisés en ce qu'ils sont constitués par les nouvelles benzothiopyrano [2,3-c] pyridines telles que définies par la formule I de la revendication 1, ainsi que par leurs sels d'addition avec les acides pharmaceutiquement acceptables.

10. Médicaments, caractérisés en ce qu'ils sont constitués par les nouvelles benzothiopyrano [2,3-c] pyridines telles que définies à la revendication 2 ou 3, ainsi que par leurs sels d'addition avec les acides pharmaceutiquement acceptables.

11. Médicaments, caractérisés en ce qu'ils sont constitués par les dérivés tels que définis à la revendication 4, ainsi que par leurs sels d'addition avec les acides pharmaceutiquement acceptables.

12. Compositions pharmaceutiques, caractérisées en ce qu'elles renferment à titre de principe actif, l'un au moins des médicaments tels que définis à l'une des revendications 9, 10 ou 11.

**Revendications** (pour l'Etat contractant AT)

1. Procédé de préparation des nouvelles benzothiopyrano [2,3-c] pyridines ainsi que de leurs sels d'addition avec les acides minéraux ou organiques, répondant à la formule générale :

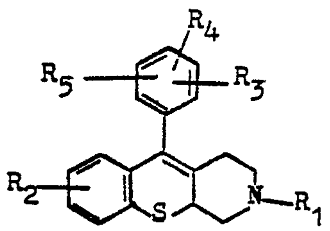

(I)

dans laquelle R$_1$ représente un atome d'hydrogène ou un radical alcoyle renfermant de 1 à 5 atomes de carbone, R$_2$ représente un atome d'hydrogène, un atome d'halogène ou un radical méthyle et R$_3$, R$_4$ et R$_5$, identiques ou différents, représentent un atome d'hydrogène, un atome d'halogène, un radical alcoyle renfermant de 1 à 5 atomes de carbone, un radical alcoxy renfermant de 1 à 3 atomes de carbone, un radical amino, un radical alcoyl ou dialcoylamino dont le radical alcoyle renferme de 1 à 3 atomes de carbone, caractérisé en ce que l'on fait réagir un organo métallique de formule :

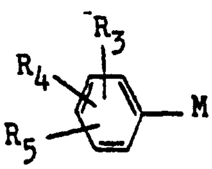

(II)

dans laquelle R$_3$, R$_4$ et R$_5$ ont la signification déjà indiquée et M représente un atome de lithium ou un radical Mg-Hal dans lequel Hal représente un atome de chlore ou de brome, ou un dérivé de cet organo-métallique, dans lequel, le ou les éventuels radicaux amino ou alkylamino sont protégés, avec un produit de formule :

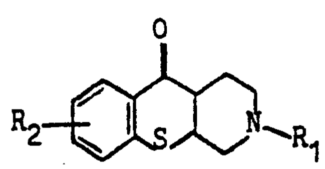

(III)

dans laquelle R$_1$ et R$_2$ ont la signification déjà indiquée, puis le cas échéant, élimine le ou les groupements protecteurs du ou des groupements amino ou alkylamino, pour obtenir un produit de formule :

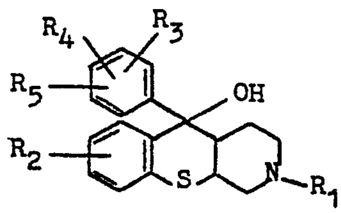

(IV)

dans laquelle $R_1$, $R_2$, $R_3$,. $R_4$ et $R_5$ ont la signification déjà indiquée, puis déshydrate ce dernier pour obtenir le produit de formule I recherché que l'on peut salifier, le cas échéant.

2. Procédé selon la revendication 1, caractérisé en ce que :

a) la réaction de l'organo métallique de formule II avec le produit de formule III est effectuée au sein d'un éther anhydre ;

b) la déshydratation du produit de formule IV est effectuée à la température d'ébullition du mélange réactionnel, au moyen d'un acide fort, ou au moyen du bisulfate de potassium, ou par chauffage dans l'hexaméthylphosphoramide.

3. Procédé selon la revendication 2, caractérisé en ce que la déshydratation est effectuée au moyen d'acide chlorhydrique ou d'acide sulfurique.

4. Procédé selon les revendications 1, 2 ou 3, caractérisé en ce que l'organo métallique de formule II, dans laquelle M représente un atome de lithium est préparé par action d'un produit de formule :

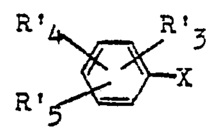

(V)

dans laquelle X représente un substituant sélectivement déplaçable par le lithium et $R'_3$, $R'_4$, et $R'_5$, identiques ou différents, représentent un atome d'hydrogène, un atome d'halogène, un radical alcoyle renfermant de 1 à 5 atomes de carbone, un radical alcoxy renfermant de 1 à 3 atomes de carbone, un radical amino ou alcoylamino protégé ou un radical dialcoylamino, les radicaux alcoyles des radicaux alcoylamino et dialcoylamino renfermant de 1 à 3 atomes de carbone, avec un alcoyl lithium suivie, si désiré, d'une hydrolyse des groupements protecteurs des radicaux amino ou alcoylamino.

5. Procédé selon la revendication 4, caractérisé en ce que l'organo-métallique de formule II est préparé *in situ*

6. Procédé selon l'une quelconque des revendications 1 à 5, caractérisé en ce que l'on utilise au départ, un produit de formule III, dans laquelle $R_1$ représente un radical méthyle.

7. Procédé selon l'une quelconque des revendications 1 à 6, caractérisé en ce que l'on utilise au départ, un produit de formule III dans laquelle $R_1$ représente un radical méthyle et $R_2$ représente un atome d'hydrogène.

8. Procédé selon l'une quelconque des revendications 1 à 5, caractérisé en ce que l'on choisit les produits de formule II et III de manière telle que l'on prépare l'un quelconque des dérivés répondant à la formule I de la revendication 1, dont les noms suivent :

— la 2-méthyl 5-phényl 1,3,4,10a-tétrahydro-2H-[1] benzothiopyrano [2,3-c] pyridine et ses sels,

— la 2-méthyl 5-(4-tolyl)1,3,4,10a-tétrahydro-2H-[1] benzothiopyrano [2,3-c] pyridine et ses sels,

— la 2-méthyl 5-(2,4-diméthylphényl)1,3,4,10a-tétrahydro-2H [1] benzothiopyrano [2,3-c] pyridine et ses sels,

— la 2-méthyl 5-(4-diméthylaminophényl)1,3,4,10a-tétrahydro-2H [1] benzothiopyrano [2,3-c] pyridine et ses sels.

**Claims** (for the Contracting States : BE, CH, DE, FR, IT, LI, LU, NL, SE)

1 New benzothiopyrano [2,3-c] pyridines as well as their salts of addition with mineral or organic acids, characterized in that they respond to the general formula :

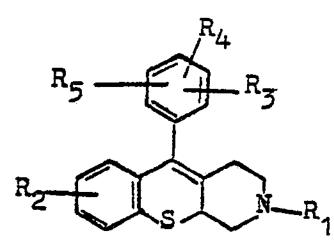

(I)

in which $R_1$ represents a hydrogen atom or an alkyl radical containing from 1 to 5 carbon atoms, $R_2$ represents a hydrogen atom, a halogen atom or a methyl radical and $R_3$, $R_4$ and $R_5$, identical or different, each represent a hydrogen atom, a halogen atom, an alkyl radical containing from 1 to 5 carbon atoms, an alkoxy radical containing from 1 to 3 carbon atoms, an amino radical, an alkyl or dialkylamino radical of which the alkyl radical contains from 1 to 3 carbon atoms.

2. Derivatives responding to formula I of claim 1 as well as their salts, characterized in that in the said formula I, $R_1$ represents a methyl radical and $R_2$, $R_3$, $R_4$ and $R_5$ have the already indicated significance.

3. Derivatives responding to formula I of claim 1 as well as their salts, characterized in that in the said

formula I, $R_1$ represents a methyl radical, $R_2$ represents a hydrogen atom and $R_3$, $R_4$ and $R_5$ have the already indicated significance.

4. Any one of the derivatives responding to formula I of claim 1, of which the names follow :

— 2-methyl 5-phenyl 1,3,4,10a-tetrahydro-2H-[1] benzothiopyrano [2,3-c] pyridine and its salts,

— 2-methyl 5-(4-tolyl) 1,3,4,10a-tetrahydro-2H-[1] benzothiopyrano [2,3-c] pyridine and its salts,

— 2-methyl 5-(2,4-dimethylphenyl) 1,3,4,10a-tetrahydro-2H [1] benzothiopyrano [2,3-c] pyridine and its salts,

— 2-methyl 5-(4-dimethylaminophenyl) 1,3,4,10a-tetrahydro-2H [1] benzothiopyrano [2,3-c] pyridine and its salts.

5. Process for the preparation of the derivatives responding to the general formula I of claim 1, as well as their salts, characterized in that an organo-metallic compound with the formula :

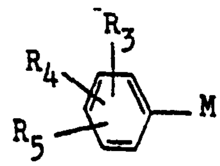

(II)

in which $R_3$, $R_4$ and $R_5$ have the significances indicated in claim 1, and M represents a lithium atom or a radical Mg-Hal in which Hal represents a chlorine or a bromine atom, or a derivative of this organo-metallic compound in which the possible amino or alkylamino radical or radicals are protected, is made to react with a product with the formula :

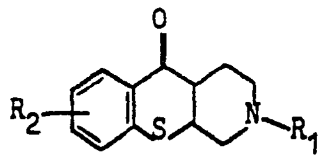

(III)

in which $R_1$ and $R_2$ have the significance indicated in claim 1, then if necessary, the protector group or groups of the amino or alkylamino group or groups are eliminated, so as to obtain a product with the formula :

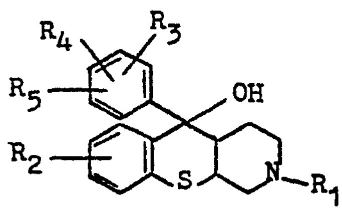

(IV)

in which $R_1$, $R_2$, $R_3$, $R_4$ and $R_5$ have the already indicated significance, then this latter is dehydrated so as to obtain the product sought with the formula I, which, if required, can be salified.

6. Process according to claim 5, characterized in that

a) the reaction of the organo-metallic compound with the formula II with the product with the formula III is carried out in an anhydrous ether.

b) the dehydratation of the product with the formula IV is carried out at the boiling point of the reactional mixture, by means of a strong acid, or by means of potassium bisulphate, or by heating in hexamethylphosphoramide.

7. Process according to claim 5, characterized in that the organo-metallic compound with the formula II, in which M represents a lithium atom is prepared by the action of a product with the formula :

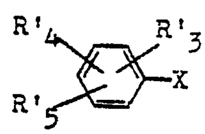

(V)

in which X represents a substituent selectively displaceable by lithium and $R'_3$, $R'_4$ and $R'_5$, identical or different, each represent a hydrogen atom, a halogen atom, an alkyl radical containing from 1 to 5 carbon atoms, an alkoxy radical containing from 1 to 3 carbon atoms, a protected amino or alkylamino radical or a dialkylamino radical, the alkyl radicals of the alkylamino and dialkylamino radicals containing from 1 to 3 carbon atoms, with a lithium alkyl followed, if desired, by a hydrolysis of the protector groups of the

amino or alkylamino radicals.

8. Process according to claim 7, characterized in that the organo-metallic compound of formula II is prepared *in situ*.

9. Medicaments, characterized in that they are constituted by the new benzothiopyrano [2,3-c] pyridines as defined by formula I of claim 1, as well as by their salts of addition with pharmaceutically acceptable acids.

10. Medicaments, characterized in that they are constituted by the new benzothiopyrano [2,3-c] pyridines as defined in claim 2 or 3, as well as by their salts of addition with pharmaceutically acceptable acids.

11. Medicaments, characterized in that they are constituted by the derivatives as defined in claim 4, as well as by their salts of addition with pharmaceutically acceptable acids.

12. Pharmaceutical compositions, characterized in that they contain, as active principle, one at least of the medicaments as defined in one of the claims 9, 10 or 11.

**Claims** (for the Contracting State AT)

1. Process for the preparation of new benzothiopyrano [2,3-c] pyridines as well as their salts of addition with mineral or organic acids, responding to the general formula :

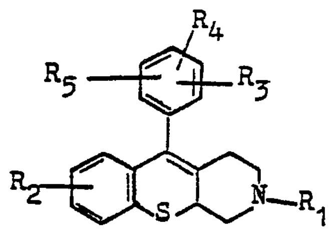

(I)

in which $R_1$ represents a hydrogen atom or an alkyl radical containing from 1 to 5 carbon atoms, $R_2$ represents a hydrogen atom, a halogen atom or a methyl radical and $R_3$, $R_4$ and $R_5$, identical or different, each represent a hydrogen atom, a halogen atom, an alkyl radical containing from 1 to 5 carbon atoms, an alkoxy radical containing from 1 to 3 carbon atoms, an amino radical, an alkyl or dialkylamino radical of which the alkyl radical contains from 1 to 3 carbon atoms, characterized in that an organo-metallic compound with the formula :

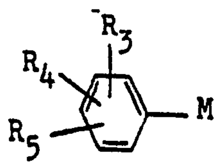

(II)

in which $R_3$, $R_4$ and $R_5$ have the significance already indicated and M represents a lithium atom or a Mg-Hal radical in which Hal represents a chlorine or a bromine atom, or a derivative of this organo-metallic compound, in which the possible amino or alkylamino radical or radicals are protected, is made to react with a product with the formula :

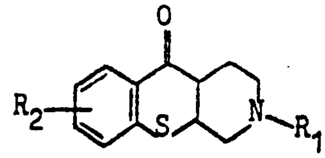

(III)

in which $R_1$ and $R_2$ have the already indicated significance, then, if required, the protector group or groups of the amino or alkylamino group or groups is/are eliminated, so as to obtain a product with the formula :

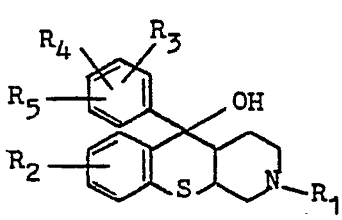

(IV)

in which $R_1$, $R_2$, $R_3$, $R_4$ and $R_5$ have the already indicated significance, then dehydrate this latter so as to obtain the product sought with the formula I, which, if required, can be salified.

2. Process according to claim 1, characterized in that

a) the reaction of the organo-metallic compound with the formula II with the product with the formula III is carried out in an anhydrous ether ;

b) the dehydratation of the product with the formula IV is carried out at the boiling point of the reactional mixture, by means of a strong acid, or by means of potassium bisulphate, or by heating in hexamethylphosphoramide.

3. Process according to claim 2, characterized in that the dehydratation is carried out by means of hydrochloric or sulphuric acid.

4. Process according to claims 1, 2 or 3, characterized in that the organo-metallic compound with the formula II, in which M represents a lithium atom is prepared by the action of a product with the formula :

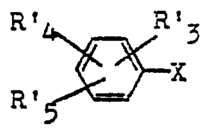

(V)

in which X represents a substituent selectively displaceable by the lithium and $R'_3$, $R'_4$, and $R'_5$, identical or different, each represent a hydrogen atom, a halogen atom, an alkyl radical containing from 1 to 5 carbon atoms, an alkoxy radical containing from 1 to 3 carbon atoms, a protected amino or alkylamino radical or a dialkylamino radical, the alkyl radicals of the alkylamino and dialkylamino radicals containing from 1 to 3 carbon atoms, with a lithium alkyl, followed, if desired, by a hydrolysis of the protector groups of the amino or alkylamino radicals.

5. Process according to claim 4, characterized in that the organo-etallic compound with the formula II is prepared *in situ*.

6. Process according to any one of the claims 1 to 5, characterized in that for starting, a product with the formula III is used, in which $R_1$ represents a methyl radical.

7. Process according to any one of the claims 1 to 6, characterized in that for starting, a product with the formula III is used, in which $R_1$ represents a methyl radical and $R_2$ represents a hydrogen atom.

8. Process according to any one of the claims 1 to 5, characterized in that the products with the formula II and III are chosen in such a way that any one of the derivatives responding to formula I of claim 1, of which the names follow, are prepared :

— 2-methyl 5-phenyl 1,3,4,10a-tetrahydro-2H-[1] benzothiopyrano [2,3-c] pyridine and its salts,

— 2-methyl 5-(4-tolyl)1,3,4,10a-tetrahydro-2H-[1] benzothiopyrano [2,3-c] pyridine and its salts,

— 2-methyl 5-(2,4-dimethylphenyl)1,3,4,10a-tetrahydro-2H [1] benzothiopyrano [2,3-c] pyridine and its salts,

— 2-methyl 5-(4-dimethylaminophenyl)1,3,4,10a-tetrahydro-2H [1] benzothiopyrano [2,3-c] pyridine and its salts.

**Ansprüche** (für die Vertragsstaaten : BE, CH, DE, FR, IT, LI, LU, NL, SE)

1. Neue Benzothiopyrano-[2,3-c]-pyridine sowie deren Additionssalze mit Mineral- oder organischen Säuren, dadurch gekennzeichnet, daß sie der allgemeinen Formel

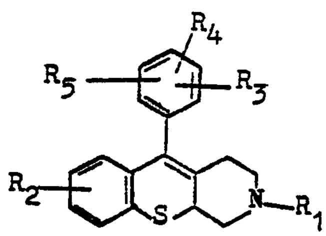

(I)

entsprechen, worin $R_1$ ein Wasserstoffatom oder einen Alkylrest mit 1 bis 5 Kohlenstoffatomen bedeutet, $R_2$ ein Wasserstoffatom, ein Halogenatom oder einen Methylrest darstellt und $R_3$, $R_4$ und $R_5$, die gleich oder verschieden sind, ein Wasserstoffatom, ein Halogenatom, einen Alkylrest mit 1 bis 5 Kohlenstoffatomen, einen Alkoxyrest mit 1 bis 3 Kohlenstoffatomen, einen Aminorest, einen Alkyl- oder Dialkylaminorest mit 1 bis 3 Kohlenstoffatomen im Alkylteil bedeuten.

2. Derivate der Formel I gemäß Anspruch 1 sowie deren Salze, dadurch gekennzeichnet, daß in der Formel I $R_1$ einen Methylrest bedeutet und $R_2$, $R_3$, $R_4$ und $R_5$ die angegebene Bedeutung besitzen.

3. Derivate der Formel I gemäß Anspruch 1 sowie deren Salze, dadurch gekennzeichnet, daß in der Formel I $R_1$ einen Methylrest darstellt, $R_2$ ein Wasserstoffatomen bedeutet und $R_3$, $R_4$ und $R_5$ die angegebene Bedeutung besitzen.

4. Derivate der Formel I gemäß Anspruch 1 mit den folgenden Bezeichnungen :
— 2-Methyl-5-phenyl-1,3,4,10a-tetrahydro-2H-[1]-benzothiopyrano-[2,3-c]-pyridin und seine Salze,
— 2-Methyl-5-(4-tolyl)-1,3,4,10a-tetrahydro-2H-[1]-benzothiopyrano-[2,3-c]-pyridin und seine Salze,
— 2-Methyl-5-(2,4-dimethylphenyl)-1,3,4,10a-tetrahydro-2H-[1]-benzothiopyrano-[2,3-c]-pyridin und seine Salze,
— 2-Methyl-5-(4-dimethylaminophenyl)-1,3,4,10a-tetrahydro-2H-[1]-benzothiopyrano-[2,3-c]-pyridin und seine Salze.

5. Verfahren zur Herstellung der Derivate der allgemeinen Formel I gemäß Anspruch 1 sowie von deren Salzen, dadurch gekennzeichnet, daß man eine metallorganische Verbindung der Formel

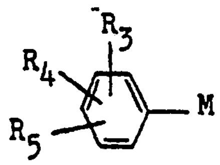

(II)

worin $R_3$, $R_4$ und $R_5$ die in Anspruch 1 angegebene Bedeutung besitzen und M ein Lithiumatom oder einen Rest Mg-Hal, worin Hal ein Chlor- oder Bromatom bedeutet, darstellt oder ein Derivat dieser metallorganischen Verbindung, worin der oder die etwaigen Amino- oder Alkylaminoreste geschützt sind, mit einem Produkt der Formel

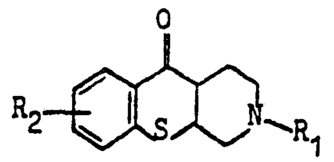

(III)

worin $R_1$ und $R_2$ die in Anspruch 1 angegebene Bedeutung besitzen, umsetzt, danach gegebenenfalls die Schutzgruppe(n) der Amino- oder Alkylaminogruppe(n) entfernt, um ein Produkt der Formel

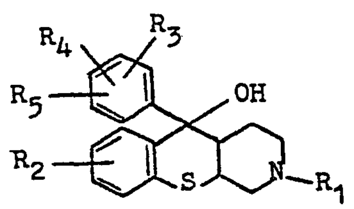

(IV)

zu erhalten, worin $R_1$, $R_2$, $R_3$, $R_4$ und $R_5$ die angegebene Bedeutung besitzen, und danach dieses letztere dehydratisiert, um das gewünschte Produkte der Formel I zu erhalten, welches man gegebenenfalls in ein Salz überführen kann.

6. Verfahren gemäß Anspruch 5, dadurch gekennzeichnet, daß

a) die Umsetzung der metallorganischen Verbindung der Formel II mit dem Produkt der Formel III in dem Medium eines wasserfreien Äthers durchgeführt wird ;

b) die Dehydratation des Produkts der Formel IV bei der Siedetemperatur des Reaktionsgemisches mit Hilfe einer starken Säure oder mit Hilfe von Kaliumbisulfat oder durch Erwärmen in Hexamethylphosphoramid durchgeführt wird.

7. Verfahren gemäß Anspruch 5, dadurch gekennzeichnet, daß die metallorganische Verbindung der Formel II, worin M ein Lithiumatom darstellt, hargestellt wird durch Umsetzen eines Produkts der Formel

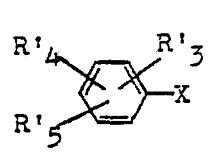

(V)

worin X einen selektiv durch Lithium austauschbaren Substituenten darstellt und $R'_3$, $R'_4$ und $R'_5$, die gleich oder verschieden sind, ein Wasserstoffatom, ein Halogenatom, einen Alkylrest mit 1 bis 5 Kohlenstoffatomen, einen Alkoxyrest mit 1 bis 3 Kohlenstoffatomen, einen geschützten Amino- oder Alkylaminorest oder einen Dialkylaminorest bedeuten, wobei die Alkylreste der Alkylamino- und Dialkylaminoreste 1 bis 3 Kohlenstoffatome enthalten, mit einer Alkyllithiumverbindung, woran sich gewünschtenfalls eine Hydrolyse der Schutzgruppen der Amino- oder Alkylaminoreste anschließt.

8. Verfahren gemäß Anspruch 7, dadurch gekennzeichnet, daß die metallorganische Verbindung der Formel II *in situ* hergestellt wird.

9. Arzneimittel, dadurch gekennzeichnet, daß sie aus den neuen Benzothiopyrano-[2,3-c]-pyridinen der Formel I gemäß Anspruch 1 oder aus deren Additionssalzen mit pharmazeutisch verträglichen Säuren bestehen.

10. Arzneimittel, dadurch gekennzeichnet, daß sie aus den neuen Benzothiopyrano-[2,3-c]-pyridinen gemäß Anspruch 2 oder 3 oder aus deren Additionssalzen mit pharmazeutisch verträglichen Säuren bestehen.

11. Arzneimittel, dadurch gekennzeichnet, daß sie aus den Derivaten gemäß Anspruch 4 oder aus deren Additionssalzen mit pharmazeutisch verträglichen Säuren bestehen.

12. Pharmazeutische Zusammensetzungen, dadurch gekennzeichnet, daß sie als Wirkstoff zumindest eines der Arzneimittel gemäß einem der Ansprüche 9, 10 oder 11 enthalten.

**Ansprüche** (für den Vertragsstaat AT)

1. Verfahren zur Herstellung von neuen Benzothiopyrano-[2,3-c]-pyridinen sowie von deren Additionssalze mit Mineral- oder organischen Säuren der allgemeinen Formel

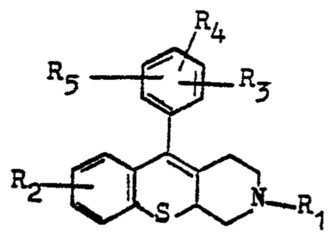

(I)

worin $R_1$ ein Wasserstoffatomen oder einen Alkylrest mit 1 bis 5 Kohlenstoffatomen bedeutet, $R_2$ ein Wasserstoffatom, ein Halogenatom oder einen Methylrest darstellt und $R_3$, $R_4$ oder $R_5$, die gleich oder verschieden sind, ein Wasserstoffatom, ein Halogenatom, einen Alkylrest mit 1 bis 5 Kohlenstoffatomen, einen Alkoxyrest mit 1 bis 3 Kohlenstoffatomen, einen Aminorest, einen Alkyl- oder Dialkylaminorest mit 1 bis 3 Kohlenstoffatomen im Alkylteil bedeuten, dadurch gekennzeichnet, daß man eine metallorganische Verbindung der Formel

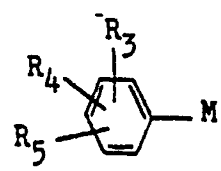

(II)

worin $R_3$, $R_4$ und $R_5$ die angegebene Bedeutung besitzen und M ein Lithiumatom oder einen Rest Mg-Hal bedeutet, worin Hal ein Chlor- oder Bromatom darstellt, oder ein Derivat dieser metallorganischen Verbindung, worin der oder die etwaigen Amino- oder Alkylaminoreste geschützt sind, mit einem Produkt der Formel

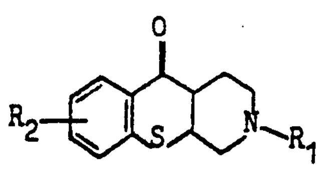

(III)

worin $R_1$ und $R_2$ die angegebene Bedeutung besitzen, umsetzt und danach gegebenenfalls die Schutzgruppe(n) der Amino- oder Alkylaminogruppe(n) entfernt, um ein Produkt der Formel

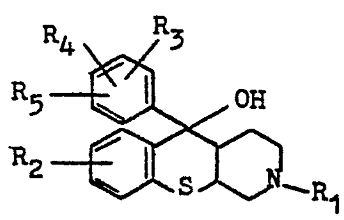

(IV)

worin $R_1$, $R_2$, $R_3$, $R_4$ und $R_5$ die angegebene Bedeutung besitzen, zu erhalten, danach dieses letztere

dehydratisiert, um das gewünschte Produkt der Formel I zu erhalten, welches man gegebenenfalls in ein Salz überführen kann.

2. Verfahren gemäß Anspruch 1, dadurch gekennzeichnet, daß

a) die Umsetzung der metallorganischen Verbindung der Formel II mit dem Produkt der Formel III in dem Medium eines wasserfreien Äthers durchgeführt wird,

b) die Dehydratation des Produkts der Formel IV bei der Siedetemperatur des Reaktionsgemisches mit Hilfe einer starken Säure oder mit Hilfe von Kaliumbisulfat oder durch Erwärmen in Hexamethylphosphoramid durchgeführt wird.

3. Verfahren gemäß Anspruch 2, dadurch gekennzeichnet, daß die Dehydratation mit Hilfe von Chlorwasserstoffsäure oder Schwefelsäure durchgeführt wird.

4. Verfahren gemäß den Ansprüchen 1, 2 oder 3, dadurch gekennzeichnet, daß die metallorganische Verbindung der Formel II, worin M ein Lithiumatom bedeutet, durch Umsetzung eines Produkts der Formel

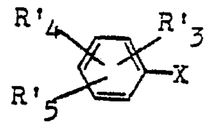

(V)

worin X einen selektiv durch Lithium austauschbaren Substituenten darstellt, und $R'_3$, $R'_4$ und $R'_5$, die gleich oder verschieden sind, ein Wasserstoffatomen, ein Halogenatom, einen Alkylrest mit 1 bis 5 Kohlenstoffatomen, einen Alkoxyrest mit 1 bis 3 Kohlenstoffatomen, einen geschützten Amino- oder Alkylaminorest oder einen Dialkylaminorest bedeuten, wobei die Alkylreste der Alkylamino- und Dialkylaminoreste 1 bis 3 Kohlenstoffatomen enthalten, mit einer Alkyllithiumverbindung hergestellt wird, woran sich gewünschtenfalls eine Hydrolyse der Schutzgruppen der Amino- oder Alkylaminoreste anschließt.

5. Verfahren gemäß Anspruch 4, dadurch gekennzeichnet, daß die metallorganische Verbindung der Formel II *in situ* hergestellt wird.

6. Verfahren gemäß einem der Ansprüche 1 bis 5, dadurch gekennzeichnet, daß man ausgeht von einem Produkt der Formel III, worin $R_1$ einen Methylrest bedeutet.

7. Verfahren gemäß einem der Ansprüche 1 bis 6, dadurch gekennzeichnet, daß man ausgeht von einem Produkt der Formel III, worin $R_1$ einen Methylrest bedeutet und $R_2$ ein Wasserstoffatom darstellt.

8. Verfahren gemäß einem der Ansprüche 1 bis 5, dadurch gekennzeichnet, daß man die Produkte der Formel II und III derart wählt, daß man eine der folgenden der Formel I gemäß Anspruch 1 entsprechenden Verbindungen mit den nachstehenden Bezeichnungen herstellt:

— 2-Methyl-5-phenyl-1,3,4,10a-tetrahydro-2H-[1]-benzothiopyrano-[2,3-c]-pyridin und seine Salze,

— 2-Methyl-5-(4-tolyl)-1,3,4,10a-tetrahydro-2H-[1]-benzothiopyrano-[2,3-c]-pyridin und seine Salze,

— 2-Methyl-5-(2,4-dimethylphenyl)-1,3,4,10a-tetrahydro-2H-[1]-benzothiopyrano-[2,3-c]-pyridin und seine Salze,

— 2-Methyl-5-(4-dimethylaminophenyl)-1,3,4,10a-tetrahydro-2H-[1]-benzothiopyrano-[2,3-c]-pyridin und seine Salze.